# EUROPEAN PATENT APPLICATION

(11) **EP 4 847 057 A1**
(43) Date of publication of application: **02.12.2026**
(21) Application number: 25754623.4
(22) Date of filing: 13.02.2025
(51) Int. Cl.: A61M 1/16, A61M 1/36

(54) **BLOOD PURIFICATION APPARATUS**

(30) Priority: 14.02.2024 JP 2024020719
(71) Applicant: Nikkiso Co., Ltd., Tokyo 150-6022 (JP)
(72) Inventor: KATAYAMA, Yuki, Higashimurayama-shi, Tokyo 1898520 (JP)
(74) Representative: von Hirschhausen, Helge
(86) International application number: PCT/JP2025/004811
(87) International publication number: WO 2025/173752

(57) **Abstract**

Provided is a blood purification apparatus capable of controlling a pump in a measurement process with an optimum parameter that is different from the one for a treatment process, and also capable of calculating the rate of recirculating blood with high accuracy. A blood purification apparatus includes a controller 16 configured to control a pump in accordance with a predetermined parameter; a characteristic-variation imparter capable of imparting a local concentration variation to a fluctuating concentration of the blood extracorporeally circulating through a blood circuit 1, the local concentration variation having a unique peak; a first detector 5a and a second detector 5b each configured to detect the local concentration variation imparted by the characteristic-variation imparter; and a recirculation-detector 17 configured to detect any recirculating blood using values detected by the first detector 5a and the second detector 5b, and to calculate a rate of the recirculating blood. In a measurement process in which the rate of the recirculating blood is calculated, the controller 16 performs a control operation after changing the parameter for activating the pump to a measurement parameter that is different from a treatment parameter for a treatment process in which blood purification treatment is performed.

## Description

### Technical Field

The present invention relates to a blood purification apparatus configured to detect any recirculating blood, which is blood reintroduced into an arterial blood circuit after being returned to a patient from a venous blood circuit.

### Background Art

In typical blood purification treatment such as hemodialysis treatment, while a patient's blood is caused to extracorporeally circulate through a blood circuit, the blood is purified by a dialyzer. However, for example, in a case where extracorporeal circulation is implemented through the patient's shunt (a site where an artery and a vein are connected to each other by a surgical operation) that is punctured with an arterial puncture needle and a venous puncture needle, recirculating blood may occur. Recirculating blood is the purified blood returned into the patient's body through the venous puncture needle but reintroduced into the blood circuit through the arterial puncture needle without flowing through the patient's organs and so forth. The occurrence of such recirculating blood means that the purified blood further undergoes extracorporeal circulation, which correspondingly reduces the amount of extracorporeal circulation of the blood that needs to be purified, leading to a problem of reduction in the blood purification efficiency.

To detect such recirculating blood, a dialysis apparatus proposed in PTL 1, for example, is capable of detecting any blood recirculation by activating an ultrafiltration pump such that a unique peak serving as a marker is imparted to the concentration of blood that fluctuates while the blood is under extracorporeal circulation. In the dialysis apparatus disclosed in PTL 1, a sensor configured to detect blood concentration (a sensor configured to detect hemoglobin concentration) is provided to the arterial blood circuit. With the detection of the unique peak by the sensor, blood recirculation that may occur during dialysis treatment can be detected.

### Citation List

### Patent Literature

PTL 1: JP2000-502940 (a Published Japanese Translation of a PCT Application)

### Summary of Invention

### Technical Problem

However, in terms of calculating the rate of recirculating blood for obtaining the recirculation rate and/or the flow rate at the patient's shunt, the known blood purification apparatus has the following problem.

In a case in which a local concentration variation having a unique peak is detected by using a first detector and a second detector, conditions for the measurement process may become unstable depending on the state of operation of the pump. Furthermore, the influence, such as pulsation, brought about by the operation of the pump included in the blood purification apparatus may hinder accurate detection of the local concentration variation. The above problem also applies to a case where the rate of recirculating blood is calculated using fluctuations in another characteristic of blood such as blood temperature, instead of blood concentration. Note that the characteristic of blood is not limited to blood concentration and encompasses all blood characteristics including blood temperature.

The present invention has been conceived in view of the above circumstances and provides a blood purification apparatus capable of controlling a pump in a measurement process with an optimum parameter that is different from the one for a treatment process, and also capable of calculating the rate of recirculating blood with high accuracy.

### Solution to Problem

According to an embodiment of the present invention, there is provided a blood purification apparatus configured to purify a patient's blood by causing the blood to extracorporeally circulate through a blood circuit including an arterial blood circuit and a venous blood circuit. The blood purification apparatus includes a controller configured to control a pump in accordance with a predetermined parameter, the pump being included in the apparatus; a characteristic-variation imparter capable of imparting a local characteristic variation to a fluctuating characteristic of the blood extracorporeally circulating through the blood circuit, the local characteristic variation having a unique peak; a first detector and a second detector provided to the arterial blood circuit and the venous blood circuit, respectively, and each being configured to detect the local characteristic variation imparted by the characteristic-variation imparter; and a recirculation-detector configured to detect any recirculating blood using the local characteristic variation detected by the first detector and the local characteristic variation detected by the second detector, and to calculate a rate of the recirculating blood, the recirculating blood being blood reintroduced into the arterial blood circuit after being returned to the patient from the venous blood circuit. In a measurement process in which the recirculation-detector detects the recirculating blood and calculates the rate of the recirculating blood, the controller performs a control operation after changing the parameter for activating the pump to a measurement parameter that is different from a treatment parameter for a treatment process in which blood purification treatment is performed.

### Advantageous Effects of Invention

According to the present invention, in the measurement process in which the recirculation-detector detects any recirculating blood and calculates the rate of the recirculating blood, the controller performs the control operation after changing the parameter for activating the pump to the measurement parameter that is different from the treatment parameter for the treatment process in which blood purification treatment is performed. That is, in the measurement process, the pump can be controlled with an optimum parameter that is different from the one for the treatment process, which enables highly accurate calculation of the rate of recirculating blood.

### Brief Description of Drawings

[Fig. 1] Fig. 1 is a diagram illustrating a blood purification apparatus according to an embodiment of the present invention.
[Fig. 2] Fig. 2 is a block diagram illustrating a main configuration of the blood purification apparatus.
[Fig. 3] Fig. 3 is a block diagram illustrating a recirculation-detector included in the blood purification apparatus.
[Fig. 4] Fig. 4 is a graph illustrating local concentration variations detected by a second detector and a first detector included in the blood purification apparatus.
[Fig. 5] Fig. 5 is a flow chart illustrating a control process that is performed by a controller included in the blood purification apparatus.

### Description of Embodiments

An embodiment of the present invention will now be described specifically with reference to the drawings.

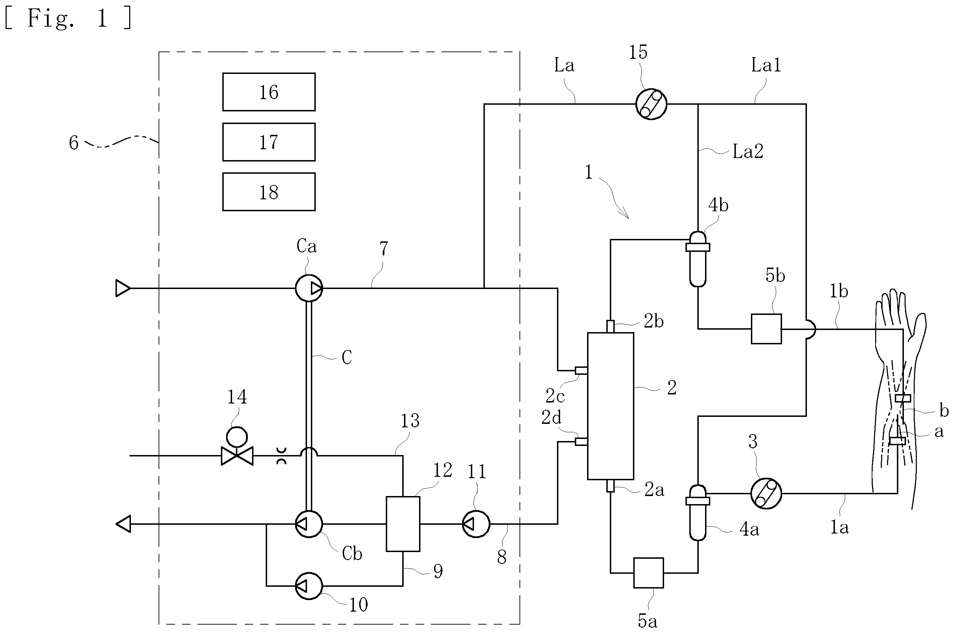

A blood purification apparatus according to the present embodiment is intended to purify a patient's blood while causing the blood to extracorporeally circulate, and is applied to a hemodialysis apparatus intended for hemodialysis treatment. As illustrated in Fig. 1, the hemodialysis apparatus includes a dialysis device 6, to which a blood circuit 1 provided with a dialyzer 2 serving as a blood purification unit is attachable. The dialysis device 6 is configured to supply dialysate to the dialyzer 2 and simultaneously perform ultrafiltration. The hemodialysis apparatus further includes a controller 16, a recirculation-detector 17, and a display 18, which are provided in the dialysis device 6.

As illustrated in the drawing, the blood circuit 1 is mainly constituted by an arterial blood circuit 1a and a venous blood circuit 1b, which are made of flexible tubes. The dialyzer 2 is connected between the arterial blood circuit 1a and the venous blood circuit 1b. The arterial blood circuit 1a is provided with an arterial puncture needle a at the distal end thereof. The arterial blood circuit 1a is further provided with a peristaltic blood pump 3, an air-trap chamber 4a for bubble removal, and a first detector 5a at respective halfway positions thereof. The venous blood circuit 1b is provided with a venous puncture needle b at the distal end thereof. The venous blood circuit 1b is further provided with a second detector 5b and an air-trap chamber 4b for bubble removal at respective halfway positions thereof.

When the blood pump 3 is activated with the patient being punctured with the arterial puncture needle a and the venous puncture needle b, the patient's blood flows through the arterial blood circuit 1a while being subjected to bubble removal at the air-trap chamber 4a and reaches the dialyzer 2, where the blood is purified. Then, the blood flows through the venous blood circuit 1b while being subjected to bubble removal at the air-trap chamber 4b and returns into the patient's body. Thus, the patient's blood is purified at the dialyzer 2 while being caused to extracorporeally circulate through the blood circuit 1.

The dialyzer 2 has a housing provided with a blood introduction port 2a, a blood delivery port 2b, a dialysate introduction port 2c, and a dialysate delivery port 2d. The proximal end of the arterial blood circuit 1a is connected to the blood introduction port 2a. The proximal end of the venous blood circuit 1b is connected to the blood delivery port 2b. The dialysate introduction port 2c and the dialysate delivery port 2d are connected to a dialysate introduction line 7 and a dialysate drain line 8, respectively. The dialysate introduction line 7 and the dialysate drain line 8 extend from the dialysis device 6.

The dialyzer 2 houses a plurality of hollow fibers. Spaces inside the respective hollow fibers serve as blood flow routes. Spaces between the inner peripheral surface of the housing and the outer peripheral surfaces of the hollow fibers serve as dialysate flow routes. The hollow fibers each have a number of microscopic holes (pores) extending therethrough from the outer peripheral surface to the inner peripheral surface, thereby forming hollow fiber membranes. Impurities and the like contained in the blood are allowed to permeate through the hollow fiber membranes into the dialysate.

The dialysis device 6 includes a duplex pump C, a bypass line 9 connected to the dialysate drain line 8 while bypassing a discharge side Cb of the duplex pump C, an ultrafiltration pump 10 connected to the bypass line 9, a pressurizing pump 11 configured to cause the dialysate to flow from the dialyzer 2 toward the discharge side Cb of the duplex pump C, a bubble isolation chamber 12, an atmosphere release line 13, and an electromagnetic valve 14.

The duplex pump C is provided astride the dialysate introduction line 7 and the dialysate drain line 8. The duplex pump C is intended to introduce the dialysate from the dialysate introduction line 7 into the dialyzer 2 and to cause the dialysate introduced into the dialyzer 2 to be drained through the dialysate drain line 8. Specifically, the duplex pump C is a metering pump, with the flow rates at a supply side Ca and the discharge side Cb thereof being substantially equal to each other. A dialysate flow route extending from the supply side Ca to the discharge side Cb (specifically, a flow route in the dialysate introduction line 7 that extends on the downstream side relative to the duplex pump C, a flow route in the dialysate drain line 8 that extends on the upstream side relative to the duplex pump C, and the dialysate flow routes in the dialyzer 2) forms a closed flow route (a flow route that is kept hermetic) when the electromagnetic valve 14 is closed.

The pressurizing pump 11 is connected to a position of the dialysate drain line 8 between the dialyzer 2 and the duplex pump C. The pressurizing pump 11 is intended to cause the dialysate to flow from the dialyzer 2 toward the duplex pump C and is a non-positive-displacement pump (a pressure-controlled type) such as a centrifugal pump. The ultrafiltration pump 10, to be described below, is also a non-positive-displacement pump (a pressure-controlled type) such as a centrifugal pump as with the pressurizing pump 11.

The dialysate introduction line 7 is connected at one end thereof to the dialysate introduction port 2c of the dialyzer 2 and at the other end thereof to a dialysate supply device (not illustrated) configured to prepare a dialysate at a predetermined concentration. The dialysate drain line 8 is connected at one end thereof to the dialysate delivery port 2d of the dialyzer 2 and at the other end thereof to draining means (not illustrated). After the dialysate supplied from the dialysate supply device flows through the dialysate introduction line 7 and reaches the dialyzer 2, the dialysate flows through the dialysate drain line 8 and the bypass line 9 and is delivered to the draining means.

The ultrafiltration pump 10 is intended to remove water from the patient's blood flowing through the dialyzer 2. Specifically, when the ultrafiltration pump 10 is activated, the amount of fluid drained through the dialysate drain line 8 becomes greater than the amount of dialysate introduced through the dialysate introduction line 7, because the duplex pump C is of a metering type. Accordingly, the excess amount of water is removed from the blood.

The bubble isolation chamber 12 is a so-called degassing chamber and has a predetermined capacity. The bubble isolation chamber 12 is connected to a position of the dialysate drain line 8 between the pressurizing pump 11 and the duplex pump C, and is capable of trapping bubbles in the dialysate. The bypass line 9 described above and the atmosphere release line 13 extend from the bubble isolation chamber 12. The distal end of the atmosphere release line 13 is open to the atmosphere. The electromagnetic valve 14, serving as valve means, is connected to a halfway position of the atmosphere release line 13.

The electromagnetic valve 14 is openable and closable to open or close the atmosphere release line 13. When the electromagnetic valve 14 is open, the bubble isolation chamber 12 communicates with outside air. When the electromagnetic valve 14 is closed, the bubble isolation chamber 12 is closed to outside air. When the atmosphere release line 13 is opened by operating the electromagnetic valve 14 before or after dialysis treatment, bubbles trapped in the bubble isolation chamber 12 are released to the atmosphere.

The dialysate introduction line 7 according to the present embodiment is provided with a supply line La. The supply line La allows dialysate serving as a substitution fluid to be supplied to the blood circuit 1. The proximal end of the supply line La is connected to a portion of the dialysate introduction line 7 between the supply side Ca of the duplex pump C and the dialysate introduction port 2c of the dialyzer 2. The distal end of the supply line La branches into a pre-substitution supply line La1 and a post-substitution supply line La2, which are connected to the air-trap chamber 4a of the arterial blood circuit 1a and the air-trap chamber 4b of the venous blood circuit 1b, respectively.

The supply line La is provided with a substitution pump 15, which is a peristaltic pump as with the blood pump 3. When the substitution pump 15 is activated during the process of blood purification treatment, the dialysate in the dialysate introduction line 7 is supplied to the arterial blood circuit 1a through the pre-substitution supply line La1 so as to enable pre-substitution, and is also supplied to the venous blood circuit 1b through the post-substitution supply line La2 so as to enable post-substitution.

The atmosphere release line 13 and the electromagnetic valve 14 according to the present embodiment constitute a characteristic-variation imparter according to the present invention. When the atmosphere release line 13 is opened by operating the electromagnetic valve 14, the blood flowing through the dialyzer 2 is concentrated abruptly and briefly, whereby a local concentration variation (characteristic variation) having a unique peak can be imparted. Specifically, during dialysis treatment, when the atmosphere release line 13 in the closed state is opened by operating the electromagnetic valve 14, the outlet pressure of the pressurizing pump 11 becomes substantially equal to the atmospheric pressure. Accordingly, a high negative pressure is instantaneously generated on the upstream side relative to the pressurizing pump 11. Consequently, the blood flowing through the dialyzer 2 (blood flow route) is subjected to abrupt and brief ultrafiltration (hemoconcentration).

Thus, the blood is subjected to ultrafiltration by a far greater amount and more briefly than with the ultrafiltration pressure that is generated by activating the ultrafiltration pump 10, and a unique peak is imparted to the fluctuating blood concentration (hematocrit value). The opening of the atmosphere release line 13 with the operation of the electromagnetic valve 14 is conducted briefly (in the present embodiment, any duration of 10 seconds or shorter may be set for the opening of the atmosphere release line 13, and satisfactory measurement accuracy is achieved even in one second). Soon after that, the electromagnetic valve 14 is operated to close the atmosphere release line 13.

It is preferable that the duration for opening the atmosphere release line 13 with the operation of the electromagnetic valve 14 be automatically optimized using blood concentration information (information on the patient's blood concentration) and/or pressure information (such as venous pressure and/or dialysate pressure). In the present invention, the phrase "abrupt and brief" refers to an extent and a duration that make the imparted pulse recognizable even after the blood circulates through the circuit. The term "unique" refers to being distinguishable from fluctuation patterns caused by any other factors such as fluctuations in the pump and a movement of the patient's body.

The first detector 5a and the second detector 5b are provided to the arterial blood circuit 1a and the venous blood circuit 1b, respectively, so as to detect the concentration (hematocrit value, specifically) of the blood flowing through the respective flow routes. For example, the first detector 5a and the second detector 5b are each a hematocrit sensor including a light-emitting device such as an LED and a light-receiving device such as a photodiode. The light-emitting device emits light toward the blood, and the light-receiving device receives the light transmitted through or reflected by the blood, whereby the hematocrit value representing the patient's blood concentration is detected.

Specifically, the first detector 5a and the second detector 5b each calculate the hematocrit value representing blood concentration from an electric signal that is output from the light-receiving device. Blood components such as red blood cells and plasma each have an inherent absorption characteristic. Using such a characteristic, the amount of red blood cells required for measuring the hematocrit value is electrooptically quantified. Thus, the hematocrit value is calculable. Near infrared rays emitted from the light-emitting device are incident on the blood and undergo absorption and scattering before being received by the light-receiving device. The rate of light absorption and scattering is analyzed from the intensity of the received rays. Thus, the hematocrit value is calculated.

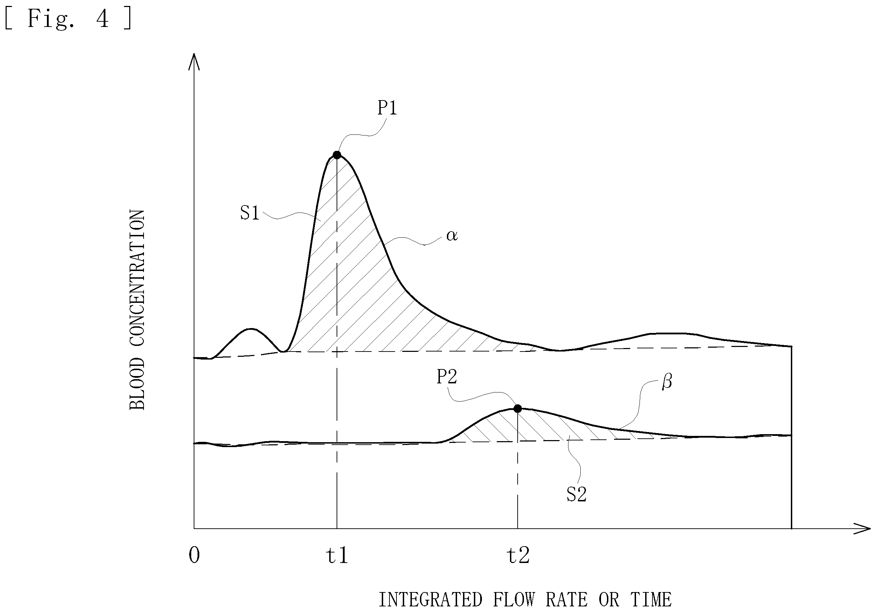

The first detector 5a configured as above is provided to the arterial blood circuit 1a and therefore detects the hematocrit value of the blood collected from the patient through the arterial puncture needle a during dialysis treatment. The second detector 5b is provided to the venous blood circuit 1b and therefore detects the hematocrit value of the blood purified by the dialyzer 2 and returning to the patient. When a local concentration variation is imparted by opening the electromagnetic valve 14, as illustrated in Fig. 4, a local concentration variation α having a unique peak P1 is detected by the second detector 5b. If such blood reaches the arterial blood circuit 1a again and undergoes recirculation, a local concentration variation β remaining in the recirculating blood and having a unique peak P2 is detected by the first detector 5a.

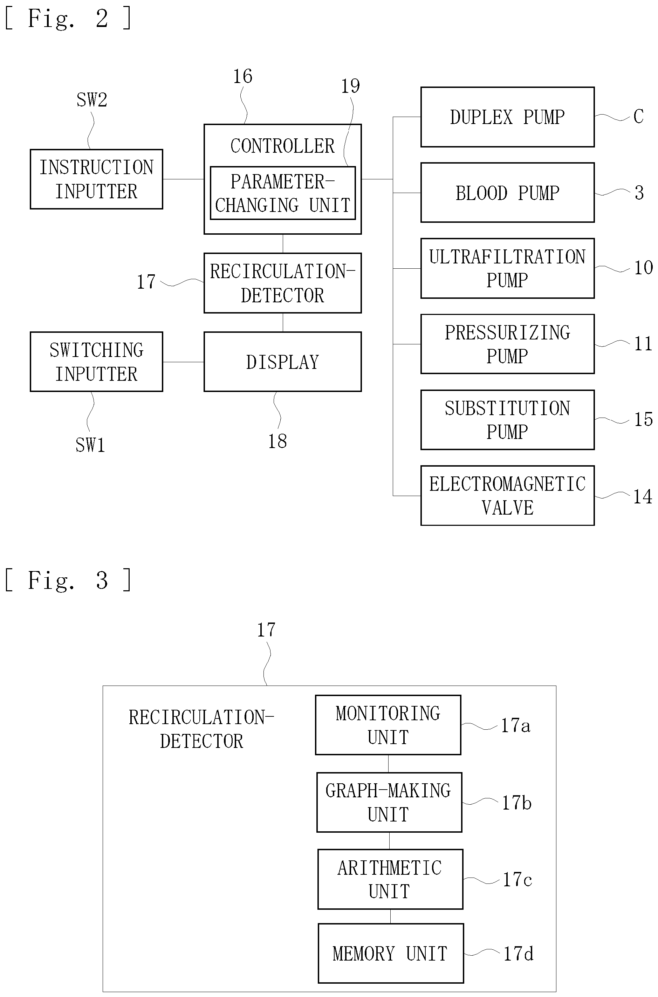

The controller 16 is, for example, a microcomputer or the like. As illustrated in Fig. 2, the controller 16 is electrically connected to and capable of controlling the following pumps and electromagnetic valves. The pumps include the duplex pump C configured to introduce and deliver dialysate into and from the dialyzer 2 (blood purification unit), the blood pump 3 provided to the blood circuit 1, the ultrafiltration pump 10 configured to remove water from the blood extracorporeally circulating through the blood circuit 1, the pressurizing pump 11, the substitution pump 15 configured to introduce the substitution fluid (dialysate) into the blood circuit, and a liquid-drug-infusing pump (not illustrated) configured to infuse a liquid drug such as heparin into the blood circuit 1. The electromagnetic valves include the electromagnetic valve 14 capable of imparting a local concentration variation having a unique peak to the fluctuating blood concentration.

As illustrated in Fig. 2, the controller 16 according to the present embodiment is connected to an instruction inputter SW2. An operator can make an input for instruction by operating the instruction inputter SW2. The instruction inputter SW2 only needs to allow the operator to make an input with any timing and may be, for example, a switch part displayed on a touch panel (including the display 18) or a mechanical switch.

The first detector 5a and the second detector 5b according to the present embodiment are electrically connected to the recirculation-detector 17 provided in the dialysis device 6. The recirculation-detector 17 is electrically connected to the display 18, which is constituted by a touch panel. The display 18 is configured to display, in the form of a graph, the local concentration variations α and β detected by the first detector 5a and the second detector 5b. In addition to displaying the graph, the display 18 is capable of displaying numerical values and/or the like representing the rate of recirculating blood and/or the like calculated by the recirculation-detector 17.

As illustrated in Fig. 2, the display 18 according to the present embodiment is connected to a switching inputter SW1. The operator can make an input for switching by operating the switching inputter SW1. The instruction inputter SW1 only needs to allow the operator to make an input with any timing and may be, for example, a switch part displayed on a touch panel (including the display 18) or a mechanical switch.

The recirculation-detector 17 is, for example, a microcomputer or the like. The recirculation-detector 17 is capable of detecting any recirculating blood using the local concentration variation β detected by the first detector 5a and the local concentration variation α detected by the second detector 5b, and calculating the rate of recirculating blood (recirculation rate). The recirculating blood is blood reintroduced into the arterial blood circuit 1a after being returned to the patient from the venous blood circuit 1b. As illustrated in Fig. 3, the recirculation-detector 17 according to the present embodiment includes a monitoring unit 17a, a graph-making unit 17b, an arithmetic unit 17c, and a memory unit 17d.

The monitoring unit 17a is configured to monitor the fluctuating blood concentrations detected by the first detector 5a and the second detector 5b on a time-course basis during blood purification treatment. The graph-making unit 17b is configured to make a graph illustrating the local concentration variations α and β from the fluctuating blood concentrations monitored on a time-course basis by the monitoring unit 17a. The arithmetic unit 17c is configured to calculate the proportion of recirculating blood (recirculation rate) using the graph made by the graph-making unit 17b. The fluctuating blood concentrations monitored by the monitoring unit 17a, the graph made by the graph-making unit 17b, and the proportion of recirculating blood (recirculation rate) calculated by the arithmetic unit 17c are stored in the memory unit 17d.

Specifically, the recirculation-detector 17 acquires fluctuations in the hematocrit values (fluctuating concentrations) detected by the first detector 5a and the second detector 5b from the graph having a horizontal axis representing time course (integrated flow rate or time) and a vertical axis representing blood concentration (hematocrit value) as illustrated in Fig. 4, and calculates an area S1 of the local concentration variation α having the unique peak P1 and an area S2 of the local concentration variation β having the unique peak P2 by a mathematical method such as integration. The proportion of recirculating blood (recirculation rate) Rrec is calculated by the following mathematical expression.$Rrec \left(%\right) = S 2 / S 1 \times 100$

The proportion of recirculating blood (recirculation rate) Rrec calculated as above is displayed on the display 18 so as to be visually recognizable by a medical worker such as a doctor. If no blood recirculation occurs, S2 in the above expression becomes 0. Accordingly, the value displayed as the proportion of recirculating blood is 0 (%). The display 18 according to the present embodiment is capable of displaying not only the recirculation rate but also the local concentration variations detected by the first detector 5a and the second detector 5b in the form of a graph as illustrated in Fig. 4. Specifically, the display 18 is capable of displaying a graph illustrating curves representing the local concentration variation α having the unique peak P1 and the local concentration variation β having the unique peak P2.

The recirculation-detector 17 is configured to make the graph illustrating the local concentration variations α and β from the blood concentrations detected by the first detector 5a and the second detector 5b, with the horizontal axis representing time course (integrated flow rate or time) and the vertical axis representing blood concentration (hematocrit value) as illustrated in Fig. 4. Furthermore, the recirculation-detector 17 is configured to monitor the fluctuating blood concentrations every predetermined period (a period of 0.1 s, for example). Such a configuration enables the detection of a start point R1 and an end point R2 of the local concentration variation α having the unique peak P1 and a start point W1 and an end point W2 of the local concentration variation β having the unique peak P2, and thus enables accurate calculation of the area S1 of the local concentration variation α and the area S2 of the local concentration variation β.

As illustrated in Fig. 2, the controller 16 according to the present embodiment includes a parameter-changing unit 19, which is a predetermined functional circuit (control circuit) or the like configured as, for example, a microcomputer. In a measurement process in which the recirculation-detector 17 detects any recirculating blood and calculates the rate of recirculating blood, the parameter-changing unit 19 performs a control operation after changing parameters for activating the pumps (the pumps to be controlled) to measurement parameters, which are different from treatment parameters for a treatment process in which blood purification treatment is performed. The pumps to be controlled by the controller 16 are various pumps included in the hemodialysis apparatus: for example, the duplex pump C, the blood pump 3, the ultrafiltration pump 10, the pressurizing pump 11, the substitution pump 15, the liquid-drug-infusing pump, and the like.

Specifically, the controller 16 is capable of controlling switching between the measurement process in which the recirculation-detector 17 detects any recirculating blood and calculates the rate of recirculating blood and the treatment process in which blood purification treatment is performed. Furthermore, in the measurement process, the controller 16 temporarily changes the parameters to the measurement parameters that are different from the parameters for the treatment process, and controls the pumps in accordance with the measurement parameters. Then, the imparting of a local concentration variation having a unique peak by the characteristic-variation imparter and the detection of any recirculating blood and calculation of the rate of recirculating blood by the recirculation-detector 17 are performed.

The measurement parameters are parameters that cause the pumps (pumps to be controlled) to operate at a lower speed than in the treatment process or to stop operating. The measurement parameters are set as specific parameters that ensure stable performance of the characteristic-variation imparter imparting a local concentration variation having a unique peak and the recirculation-detector 17 detecting any recirculating blood and calculating the rate of recirculating blood in the measurement process.

In particular, the controller 16 according to the present embodiment temporarily changes the parameters for activating the pumps to the measurement parameters, activates the pumps in accordance with the measurement parameters, and waits until the blood concentrations detected by the first detector 5a and the second detector 5b are stabilized. Furthermore, on condition that the blood concentrations are stabilized, the imparting of a local concentration variation having a unique peak by the characteristic-variation imparter and the detection of any recirculating blood and calculation of the rate of recirculating blood by the recirculation-detector are performed.

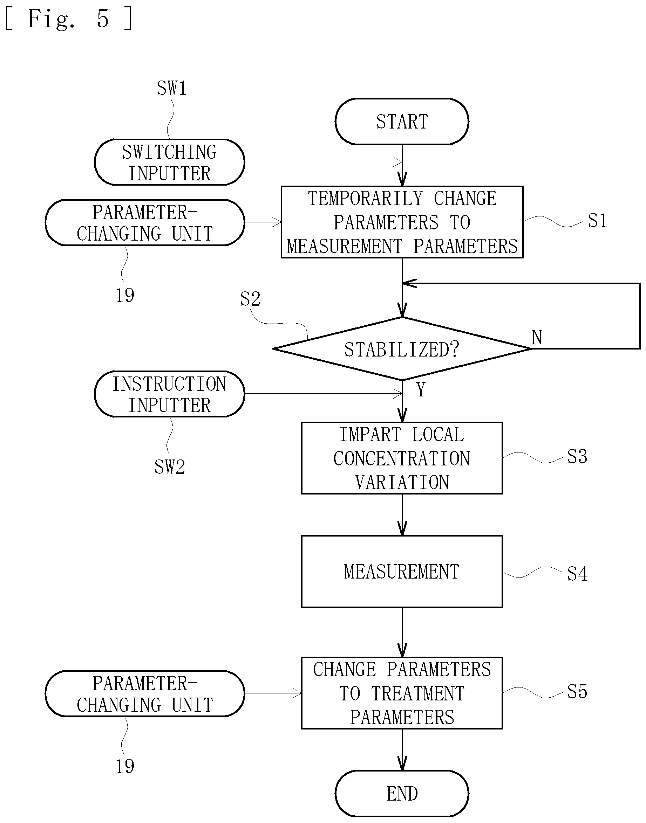

Thus, in the present embodiment, the process in which the recirculation-detector 17 detects any recirculating blood and calculates the rate of recirculating blood is performed in the measurement process that is different from the treatment process in which blood purification treatment is performed. The measurement process will now be described with reference to a flow chart illustrated in Fig. 5.

First, the measurement process is started on condition that an input operation is performed on the switching inputter SW1 by an operator. In response to this, in step S1, the parameter-changing unit 19 of the controller 16 temporarily changes the parameters of the pumps to be controlled to the measurement parameters, and activates the pumps in accordance with the measurement parameters. Then, in step S2, the controller 16 waits until the blood concentrations detected by the first detector 5a and the second detector 5b are stabilized. The measurement parameters are parameters that cause the pumps (pumps to be controlled) to operate at a lower speed than in the treatment process. After the blood concentrations detected by the first detector 5a and the second detector 5b are stabilized, the process proceeds to step S3 on condition that an input operation is performed on the instruction inputter SW2 by the operator. In step S3, the atmosphere release line 13 is opened by operating the electromagnetic valve 14, whereby a local concentration variation that forms unique peaks P1 and P2 is imparted.

Subsequently, the process proceeds to step S4, where the recirculation-detector 17 detects any recirculating blood using a local concentration variation α detected by the second detector 5b and a local concentration variation β detected by the first detector 5a, and calculates the rate of recirculating blood (recirculation rate). The rate of recirculating blood (recirculation rate) thus calculated is displayed on the display 18 so as to prompt a medical worker such as a doctor to take an appropriate action. After any recirculating blood is detected and the rate of recirculating blood (recirculation rate) is calculated, the process proceeds to step S5, where the parameter-changing unit 19 of the controller 16 changes the measurement parameters to the treatment parameters. Thus, the measurement process ends, and blood purification treatment is allowed to be performed in accordance with the treatment parameters.

According to the present embodiment, in the measurement process in which the recirculation-detector 17 detects any recirculating blood and calculates the rate of recirculating blood, the controller 16 performs a control operation after changing the parameters for activating the pumps to the measurement parameters that are different from the treatment parameters for the treatment process in which blood purification treatment is performed. That is, in the measurement process, the pumps are controlled with optimum parameters that are different from those for the treatment process, which enables highly accurate calculation of the rate of recirculating blood.

While an embodiment has been described above, the present invention is not limited thereto. For example, the characteristic-variation imparter is not limited to the one constituted by the atmosphere release line 13 and the electromagnetic valve 14 and may be any other means, as long as the characteristic-variation imparter is capable of imparting a local concentration variation having a unique peak. The characteristic-variation imparter according to the above embodiment is configured to concentrate the blood through ultrafiltration so as to make a local variation in blood concentration. Alternatively, for example, the local variation in blood concentration may be made by introducing a fluid such as a substitution fluid (dialysate) into the blood circuit so as to dilute the blood.

The first detector 5a and the second detector 5b may each be any device (such as a sensor configured to detect the concentration of hemoglobin, or a sensor configured to detect the concentration of protein or the like) other than a hematocrit sensor, as long as the device is capable of detecting fluctuating blood concentration having unique peaks. The first detector 5a and the second detector 5b may each be provided at any position of a corresponding one of the arterial blood circuit 1a and the venous blood circuit 1b.

While the fluctuating characteristic of blood according to the present embodiment is the fluctuating concentration of blood, the fluctuating characteristic of blood may be another fluctuating characteristic such as blood temperature. For example, ultrasonic waves may be used as follows: with a marker (such as physiological saline or microbubbles) infused into the blood, the curve of dilution may be measured with an ultrasonic probe to observe changes in bloodstream in the blood circuit on a real-time basis, whereby the recirculation rate may be quantified.

As another example, with a pigment infused into the arterial blood circuit, the light absorbance in the venous blood circuit may be measured to evaluate the occurrence of recirculation. As yet another example, with cooled physiological saline infused into the blood circuit, changes in blood temperature may be measured with temperature sensors provided to the arterial blood circuit and the venous blood circuit, whereby the recirculation rate may be calculated from the rate of change in the temperature. As yet another example, electrical impedance may be used as follows: changes in the electrical impedance of the blood in the blood circuit may be measured to analyze changes in the flow of blood, and a change in the impedance that is attributed to recirculation may be calculated using the electric conductivity of the physiological saline. As yet another example, optics may be used as follows: changes in the oxygen saturation of the blood or hemoglobin concentration may be measured by infrared spectroscopy (NIRS), and data for the arterial blood circuit and data for the venous blood circuit may be compared to evaluate the influence of recirculation.

According to a first aspect of the present invention, there is provided a blood purification apparatus configured to purify a patient's blood by causing the blood to extracorporeally circulate through a blood circuit 1 including an arterial blood circuit 1a and a venous blood circuit 1b. The blood purification apparatus includes a controller 16 configured to control a pump in accordance with a predetermined parameter, the pump being included in the apparatus; a characteristic-variation imparter capable of imparting a local concentration variation to a fluctuating concentration of the blood extracorporeally circulating through the blood circuit 1, the local concentration variation having a unique peak; a first detector 5a and a second detector 5b provided to the arterial blood circuit 1a and the venous blood circuit 1b, respectively, and each being configured to detect the local concentration variation imparted by the characteristic-variation imparter; and a recirculation-detector 17 configured to detect any recirculating blood using the local concentration variation β detected by the first detector 5a and the local concentration variation α detected by the second detector 5b, and to calculate a rate of the recirculating blood, the recirculating blood being blood reintroduced into the arterial blood circuit 1a after being returned to the patient from the venous blood circuit 1b. In a measurement process in which the recirculation-detector 17 detects the recirculating blood and calculates the rate of the recirculating blood, the controller 16 performs a control operation after changing the parameter for activating the pump to a measurement parameter that is different from a treatment parameter for a treatment process in which blood purification treatment is performed. That is, in the measurement process, the pump can be controlled with an optimum parameter that is different from the one for the treatment process, which enables highly accurate calculation of the rate of recirculating blood.

According to a second aspect of the present invention, in the first aspect, the measurement parameter is a parameter that causes the pump to operate at a lower speed than in the treatment process or to stop operating. That is, a specific parameter can be set that ensures stable performance of the characteristic-variation imparter imparting a local concentration variation having a unique peak and the recirculation-detector 17 detecting any recirculating blood and calculating the rate of recirculating blood in the measurement process.

According to a third aspect of the present invention, in the first aspect, the controller 16 temporarily changes the parameter for activating the pump to the measurement parameter, activates the pump in accordance with the measurement parameter, and waits until the blood concentrations detected by the first detector 5a and the second detector 5b are stabilized. Furthermore, on condition that the blood concentrations are stabilized, the imparting of the local concentration variation having the unique peak by the characteristic-variation imparter and the detection of the recirculating blood and the calculation of the rate of the recirculating blood by the recirculation-detector are performed. That is, the imparting of a local concentration variation having a unique peak by the characteristic-variation imparter can always be performed in a stable condition, which enables the recirculation-detector to achieve accurate performance of the detection of any recirculating blood and the calculation of the rate of recirculating blood.

According to a fourth aspect of the present invention, in the third aspect, after the detection of the recirculating blood and the calculation of the rate of the recirculating blood by the recirculation-detector are performed, the controller automatically changes the parameter for activating the pump to the treatment parameter. That is, after the measurement process ends, a smooth transition to the treatment process can be achieved.

According to a fifth aspect of the present invention, in the first aspect, the characteristic of the blood is blood concentration. That is, the rate of recirculating blood can be calculated by using fluctuating blood concentration in detecting the recirculating blood.

### Industrial Applicability

The present invention is also applicable to any blood purification apparatus having a different appearance, additional functions, or the like within the spirit of the present invention.

### Reference Signs List

- 1: blood circuit
- 1a: arterial blood circuit
- 1b: venous blood circuit
- 2: dialyzer (blood purification unit)
- 3: blood pump
- 4a, 4b: air-trap chamber
- 5a: first detector
- 5b: second detector
- 6: dialysis device
- 7: dialysate introduction line
- 8: dialysate drain line
- 9: bypass line
- 10: ultrafiltration pump
- 11: pressurizing pump
- 12: bubble isolation chamber
- 13: atmosphere release line
- 14: electromagnetic valve
- 15: substitution pump
- 16: controller
- 17: recirculation-detector
- 18: display
- C: duplex pump
- La: supply line
- La1: pre-substitution supply line
- La2: post-substitution supply line
- SW1: switching inputter
- SW2: instruction inputter

## Claims

1. A blood purification apparatus configured to purify a patient's blood by causing the blood to extracorporeally circulate through a blood circuit including an arterial blood circuit and a venous blood circuit, the blood purification apparatus comprising:
a controller configured to control a pump in accordance with a predetermined parameter, the pump being included in the apparatus;
a characteristic-variation imparter capable of imparting a local characteristic variation to a fluctuating characteristic of the blood extracorporeally circulating through the blood circuit, the local characteristic variation having a unique peak;
a first detector and a second detector provided to the arterial blood circuit and the venous blood circuit, respectively, and each being configured to detect the local characteristic variation imparted by the characteristic-variation imparter; and
a recirculation-detector configured to detect any recirculating blood using the local characteristic variation detected by the first detector and the local characteristic variation detected by the second detector, and to calculate a rate of the recirculating blood, the recirculating blood being blood reintroduced into the arterial blood circuit after being returned to the patient from the venous blood circuit,
wherein, in a measurement process in which the recirculation-detector detects the recirculating blood and calculates the rate of the recirculating blood, the controller performs a control operation after changing the parameter for activating the pump to a measurement parameter that is different from a treatment parameter for a treatment process in which blood purification treatment is performed.

2. The blood purification apparatus according to claim 1, wherein the measurement parameter is a parameter that causes the pump to operate at a lower speed than in the treatment process or to stop operating.

3. The blood purification apparatus according to claim 1, wherein the controller temporarily changes the parameter for activating the pump to the measurement parameter, activates the pump in accordance with the measurement parameter, and waits until the blood characteristics detected by the first detector and the second detector are stabilized, and wherein, on condition that the blood characteristics are stabilized, the imparting of the local characteristic variation having the unique peak by the characteristic-variation imparter and the detection of the recirculating blood and the calculation of the rate of the recirculating blood by the recirculation-detector are performed.

4. The blood purification apparatus according to claim 3, wherein after the detection of the recirculating blood and the calculation of the rate of the recirculating blood by the recirculation-detector are performed, the controller automatically changes the parameter for activating the pump to the treatment parameter.

5. The blood purification apparatus according to claim 1, wherein the characteristic of the blood is blood concentration.
